# EUROPEAN PATENT APPLICATION

(11) **EP 3 409 137 A1**
(43) Date of publication of application: **05.12.2018**
(21) Application number: 17173936.0
(22) Date of filing: 01.06.2017
(51) Int. Cl.: A43B 7/14, A61F 5/01, A61F 5/14

(54) **INSOLE WITH AN INTEGRATED ELASTIC ELEMENT**

(71) Applicant: M.G. Fitmess S.r.l., 25136 Brescia (IT)
(72) Inventor: BERTRAND, Gennaro, 41122 MODENA (IT)
(74) Representative: Gagliardelli, Fabrizio

(57) **Abstract**

A deformable insole (1) for footwear defining an upper surface (2) adapted in use to face towards the sole of a user's foot, and a lower surface (3) adapted in use to face towards the sole of the footwear.

The insole comprises a housing (4) interposed between a sector of the upper surface and a sector of the lower surface, the housing (4) being suitable for containing an elastic element (5) and holding it firmly between the sectors.

## Description

The present invention relates to a deformable insole suitable for being inserted in footwear, particularly for the correction of postural defects.

At present, insoles exist which serve to dampen the user's movement of resting each foot on the ground, during which a certain portion of the user's weight is applied.

By using a pair of insoles for the respective feet, it is possible to compensate for any imbalances in the distribution of the user's weight between the two lower limbs during walking.

This compensation can also be obtained by means of insoles which exhibit a marked flexural deformability during walking.

In particular, the latter are conformed in such a way as to rest on the sole in a rear portion and a front portion of the insole, so that the central portion is raised and can move up and down, relative to the sole, in an alternating fashion during walking.

In this way, the insole can deform flexurally more or less proportionally to the weight applied by the respective foot of the user.

Although such insoles are effective in correcting postural defects, they are not suitable for the practice of sports, i.e. they do not allow good enjoyment of sports activity on the part of those who suffer from the aforementioned postural problems.

The object of the invention is to overcome the limits of the prior art by providing an insole for postural correction which can be used in an effective manner during sports activity or, in general, during physical activity.

This object is achieved by the insole made in accordance with claim 1.

The features of the present invention shall become clear from a reading of the following detailed description, which is offered by way of example and does not limit the more general features claimed.

The following detailed description refers to the attached figures, in which:
- figure 1 is a perspective view of an insole according to a possible embodiment of the present invention in a first operative condition;
- figure 2 is a perspective view of an insole according to that embodiment in a second operative condition;
- figure 3 is a top view of the insole of figure 1;
- figure 4 is a bottom view of the insole of figure 1;
- figure 5 is a side view of the insole of figure 1;
- figure 6 is a view of the B-B cross section of figure 3.

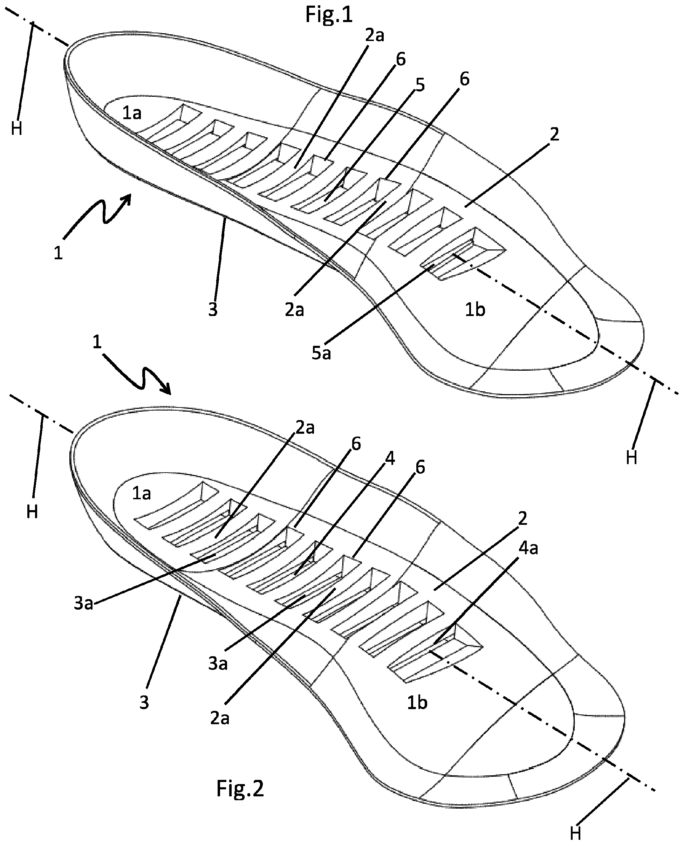

Figure 1 shows an insole 1 according to a possible embodiment of the present invention. The insole 1 defines an upper surface 2 adapted in use to face towards the sole of a user's foot, and a lower surface 3 adapted in use to face towards the sole of the footwear.

The upper surface 2 is therefore the one that, under a normal condition of use, is directly pressed by the user's foot, at least when the respective foot is resting on the ground. The lower surface 3 is the one that, under such a condition of use, is usually at least partially in contact with the sole of the footwear.

The insole 1 comprises a housing 4. The housing 4 is interposed between at least one sector of said upper surface 2 and at least one sector of said lower surface 3.

Sector of the upper surface 2 or of the lower surface 3 means a part of that respective surface, as seen, for example, in a plan view.

The housing 4 is suitable for containing an elastic element 5 and holding it firmly between said sectors, so that said elastic element 5 will remain in the housing 4 while the user is walking or running and thus influence the deformability and/or structural behaviour and/or elasticity of the insole 1.

In figure 1 the insole 1 is shown in an operative condition in which the elastic element 5 is inside the housing 4. Figure 2 shows another operative condition of the insole 1, in which the housing 4 is empty, and thus without the elastic element.

The insole 1 could also be used in the condition of figure 2.

In figure 1 the elastic element 5 is indicated, whereas in figure 2 the housing 4 is indicated, more or less where the elastic element 5 is indicated in figure 1, since in figure 2 the elastic element is not present.

In order to clarify the positioning of the elastic element 5, in figure 1 an end 5a of the elastic element 5 is also indicated.

The elastic element 5 preferably has a shape at least partially complementary to that of the housing 4, and/or a shape at least partially mating that of the housing 4. The elastic element 5 has a preferably elongated shape, and, in the embodiment shown, can be defined as a tongue.

The elastic element 5 is thus integrated in the insole 1 in the condition of figure 1, but can be removed and replaced with another elastic element 5 with different structural characteristics, for example more flexible, or alternatively more rigid, than the previous one, and/or made of another material.

A given user could in fact decide to use a more rigid insole 1, for example when more accustomed to the physical activity he must engage in, and in such a case he will choose a more rigid elastic element 5.

A user who decides instead to have a greater dampening effect from the insole 1 will choose an elastic element 5 that is more flexible or elastic.

The elastic element 5 can be advantageously made up at least partially of a composite material, which for example can comprise carbon fibres embedded in an epoxy or other type of matrix as a so-called "reinforcement".

Depending on the choice of reinforcement and the density thereof, elastic elements of different rigidity will be obtained.

Preferably, the housing 4 is configured to allow the insertion of the elastic element 5 into the housing 4 itself, and the removal or extraction thereof from the housing 4, along a direction parallel to said sector of the upper surface 2 and/or said sector of the lower surface 3.

The insole 1 shown in the figures defines a longitudinal axis H, which is oriented from a rear portion 1 a of the insole 1 towards a front portion 1b of the insole 1, or vice versa.

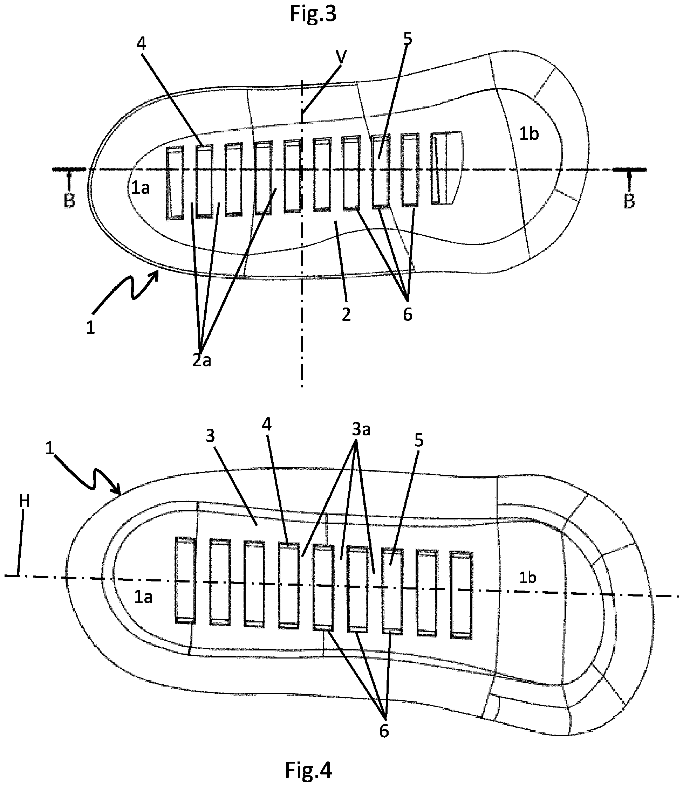

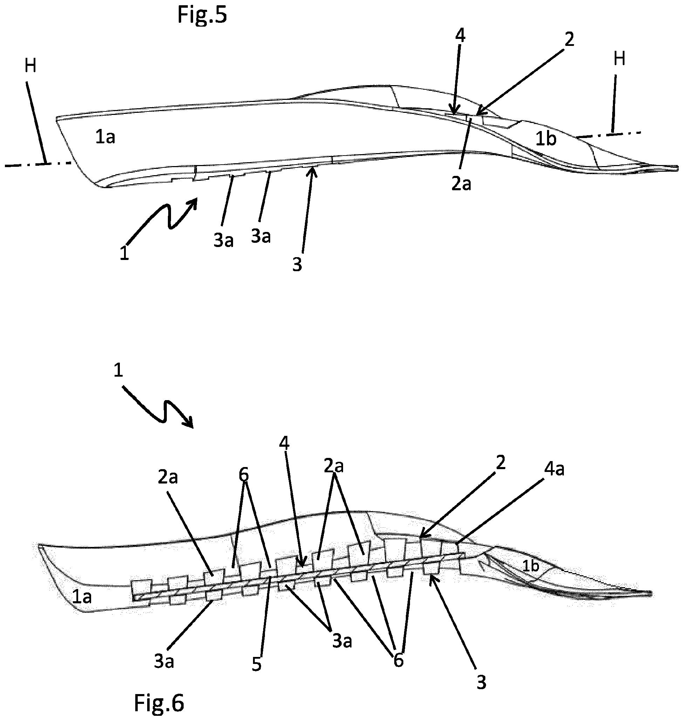

The positioning of the front portion 1 b and the rear portion 1 a of the insole, is visible from above in figure 3, from below in figure 4, and from the side in figures 5 and 6.

In a condition of normal use, the front portion 1 b of the insole 1 is adapted to be pressed respectively by the front part of a user's foot, relative to a direction of forward movement of the user during normal walking or running.

In such a condition of use, the rear portion 1 a of the insole 1 is adapted to be pressed respectively by the rear part of a user's foot.

The longitudinal axis H is indicated only in figures 1-2, 4 and 5, for the sake of clarity of the other figures.

The insole 1 shown in the figures defines a transverse axis V orthogonal to said longitudinal axis H. The transverse axis V is indicated only in figure 3, for the sake of clarity of the figures.

The housing 4 is indicated also in figures 3 and 4, given that, in said figures 3 and 4, despite the elastic element 5 being present, the same elastic element 5 does not occupy, along the transverse axis V, all the space of the housing 4.

The housing 4 is configured to allow the insertion of the elastic element 5 into the housing 4 itself, and the removal or extraction thereof from the housing 4, mainly along a direction parallel to the longitudinal axis H.

The direction can coincide with the longitudinal axis H.

The housing 4 has at least one opening 4a for the entry of the elastic element 5 into the housing 4 and for the exit of the elastic element 5 from the housing 4. The opening 4a is disposed transversely to said longitudinal axis H, so as to allow the entry of the elastic element 5 into the housing 4, and the exit of the elastic element 5 from the housing 4 starting from said opening 4a. The opening 4a is oriented and/or turned and/or disposed in order that the entry and exit can take place mainly in a direction parallel to the longitudinal axis H.

This direction can coincide with the longitudinal axis H.

Advantageously, the housing 4 of the insole 1 shown in the figures mainly extends along an axis of extension parallel to said longitudinal axis H. The axis of extension can coincide with the longitudinal axis H.

In this way, the insertion of the elastic element 5 into the housing 4 and the removal thereof from the housing 4 are facilitated.

Advantageously, the housing 4 mainly extends along the direction along which the entry or insertion of the elastic element 5 into the housing 4 and the exit or removal of the elastic element 5 from the housing 4 can take place.

As said above, the housing 4 is interposed or situated between a sector of the upper surface 2 and a sector of the lower surface 3 of the insole 1. The insole 1 defines at least one of said sectors by means of a plurality of bands, upper 2a or lower 3a, respectively.

The upper bands 2a or lower bands 3a are at least partially separated from one another.

The upper bands 2a or lower bands 3a are preferably separated from one another at the housing 4.

The upper bands 2a and the lower bands 3a delimit or define the housing 4 from respective sides reciprocally opposed relative to an axis aligned with the direction of insertion and removal of the elastic element 5, or relative to the axis of extension of the housing 4.

At least one, and preferably each, of the upper bands 2a or lower bands 3a, is elongated and extends along the transverse axis V of the insole 1, in such a way as to weaken the insole 1 relative to at least the flexion of the same insole 1. This flexion can be considered for example associated with the bending of the insole 1 on a plane containing the longitudinal axis H and parallel to the figures 5 and 6.

The bands 2a or 3a are advantageously separated or spaced apart from one another by slots 6, or by slits or apertures.

The slots 6 indicated in figures 1 - 4 are identified by an indication of one of the respective edges for each slot 6 that is indicated. In figures 5 and 6 the slots 6 are identified by an indication of the respective empty space between the bands 2a or 3a, which delimit each respective slot 6 that is indicated.

It should be borne in mind that, for the sake of clarity, only some of the bands 2a or 3a, or slots 6, are indicated in the appended figures; they are visible in each respective figure.

The bands 3a situated on the side of the lower surface 3 are not indicated in figure 1, as they are not visible due to the presence of the elastic element 5.

In the embodiment shown, both sectors of the upper surface and lower surface, which delimit the housing 4 at the top and bottom, and thus both those of the upper surface 2 and lower surface 3, are defined by a respective plurality of bands 2a or 3a.

In a possible embodiment of the invention, not represented, only the aforesaid sector of the lower surface 3 is endowed with bands and slots, whereas the upper surface is continuous, wholly to the advantage of comfort in the use of the insole 1.

Preferably, the rear portion of the insole 1, in the area accommodating the heel of the foot, is thicker than a front portion, so as to provide greater support precisely at the heel.

Furthermore, it should be noted that the insole of the invention is conceived so as to be able to deform according to the user's weight; therefore, the greater the weight, the greater the deformation during use. According to a possible embodiment of the invention, the elastic element 5 could also be permanently housed in the housing 4.

The invention achieves the proposed objects, and makes it possible to obtain a highly versatile insole, which can be used by users with different builds and/or postures and a different weight, and is simultaneously effective in use during physical and sports activity.

## Claims

1. A deformable insole (1) for footwear defining an upper surface (2) adapted in use to face towards the sole of a user's foot, and a lower surface (3) adapted in use to face towards the sole of the footwear, **characterized in that** it comprises a housing (4) interposed between a sector of said upper surface (2) and a sector of said lower surface (3), said housing (4) being suitable to contain an elastic element (5) and to hold it firmly between said sectors.

2. An insole (1) according to claim 1, wherein said insole (1) comprises said elastic element (5), said elastic element (5) being housed permanently in said housing (4).

3. An insole (1) according to claim 1, wherein said housing (4) is suitable for allowing removal of said elastic element (5) and replacement thereof.

4. An insole (1) according to claim 3, wherein said housing (4) is so configured as to allow insertion of said elastic element (5) into said housing (4) and removal or extraction thereof from the housing (4) itself along an insertion and extraction direction, said direction being parallel to said sectors and/or being parallel to a longitudinal axis (H) of the insole (1).

5. An insole (1) according to claim 4, wherein said housing (4) exhibits at least one opening (4a) in order that said elastic element (5) can be inserted into said housing (4), and that said elastic element (5) can be removed from said housing (4), said opening (4a) being arranged transversely to said longitudinal axis (H).

6. An insole (1) according to any preceding claim, wherein said insole (1) defines at least one of said sectors by a plurality of bands (2a; 3a) separated from one another.

7. An insole (1) according to claim 6, wherein said bands (2a, 3a) are separated or spaced apart from one another by slots (6) or slits.

8. An insole (1) according to claim 6 or 7, wherein each of said bands (2a, 3a) is elongated and develops along a transverse axis (V) of the insole.

9. An insole (1) according to claim 8, wherein said insole (1) comprises an arcuate central portion interposed between a front portion (1b) and a rear portion (1a) of the insole (1), and said housing (4) is arranged within said central portion.

10. An insole (1) according to claim 9, wherein said central portion is raised such that said front portion (1 b) and rear portion (1 a) define respectively the lowered portions of the insole (1).

11. An insole (1) according to any preceding claim, in which said elastic element (5) is made up at least partially of carbon fiber.
